# EUROPEAN PATENT APPLICATION

(11) **EP 3 563 844 A1**
(43) Date of publication of application: **06.11.2019**
(21) Application number: 18209803.8
(22) Date of filing: 03.12.2018
(51) Int. Cl.: A61K 31/375, A61K 9/00, A61P 31/04, A61P 43/00, A23L 2/00, C05B 3/00

(54) **COMPOSITIONS COMPRISING A BUFFER AND A UREASE ACTIVITY INHIBITOR**

(30) Priority: 30.04.2018 TW 10714754
(71) Applicant: Eco-Geo Bio-Technology Company Limited, 10694 Taipei City (TW)
(72) Inventor: Shen, Ta-Lu, 10694 Taipei City (TW); Chen, Fu-An, 10694 Taipei City (TW)
(74) Representative: Isarpatent

(57) **Abstract**

A composition for inhibiting the activity of a urease is disclosed, which includes at least one acid component, at least one base component and a urease activity inhibitor, wherein the at least one acid component is one of an organic acid, a phosphoric acid and a combination thereof, the at least one base component is one of an organic base, a phosphate and a combination thereof, wherein the at least one acid component and the at least one base component conjugate with each other to form a buffer formulation, and the urease activity inhibitor is one of an ascorbic acid, a salt of the ascorbic acid and a combination thereof, wherein the buffer formulation steadily sustains the activity of the urease activity inhibitor.

## Description

### FIELD OF THE INVENTION

The present invention relates to a composition for inhibiting the activity of ureases in a human being, an animal or the soil, specifically to a use and a composition for inhibiting the activity of ureases in a broad pH range across acid and base.

### BACKGROUND OF THE INVENTION

Ureases have a high degree of homology among different bacterium, and they hydrolyze urea to ammonia and carbon dioxide. Ureases are commonly found in bacterial infections in the gastrointestinal tract of the human and the animal, and it is a virulence factor for some pathogens in humans and animals.

Ureases can be divided into two classes including cytoplasmic urease and surface urease, and pathogens with both kinds of ureases have a better resistance to acid exposure while pathogens with the cytoplasmic urease only are more susceptible to acid (Helicobacter pylori: Physiology and Genetics(2001)).

*Helicobacter pylori* is a parasitic bacteria in the human digestive tract, and it usually exists in the gastric mucosal epithelium but can also proliferate in the oral cavity. The urease of *Helicobacter pylori* can promptly break down urea to ammonia and carbon dioxide so as to make *Helicobacter pylori* less susceptible to gastric acid attack in the stomach.

Urea is a main nitrogenous fertilizer that includes about 46% of nitrogen. When urea fertilizer is administered to the soil, the urease facilitates a rapid hydrolysis of the urea to generate volatile ammonia (i.e. a source of environmental pollution).

In view of the above-mentioned problems caused by urease, the Applicant of the present application has developed a non-drug inhibitor for inhibiting the activity of urease in humans, animals or the soil, and the non-drug inhibitor compensates for the defects of the existing products. The summary of the present invention is described below.

### SUMMARY OF THE INVENTION

The main object of the present invention is to provide a composition for inhibiting the activity of urease in the gastrointestinal tract of humans or non-human animals or in a soil. In order not to disturb the normal physiology of the gastrointestinal tract, a multiple pHs buffer formulation composition that operates optimally under different pH conditions of the digestive tract is provided in the present invention, and thereby the activity of the urease is inhibited.

The multiple pHs buffer formulation composition of the present invention can adapt to various pH environments in the gastrointestinal tract, inhibit the activity of urease under an environment across acid and base (pH 5-8), and significantly inhibit the activity of urease at pH 6.8-7.3. To achieve the above-mentioned effects, the multiple pHs buffer formulation composition of the present invention includes at least one acid component, at least one base component and a urease activity inhibitor, wherein the at least one acid component is one selected from a group consisting of an organic acid, a phosphoric acid and a combination thereof, the at least one base component is one selected from a group consisting of an organic base, a phosphate and a combination thereof, and the urease activity inhibitor is one selected from a group consisting of an ascorbic acid, a salt of the ascorbic acid and a combination thereof. The acid component and the base component form a buffer formulation so as to steadily sustain the activity of the urease activity inhibitor in the buffer formulation.

The present invention provides a composition for inhibiting the activity of a urease, which includes at least one acid component, at least one base component and a urease activity inhibitor, wherein the at least one acid component is one selected from a group consisting of an organic acid, a phosphoric acid and a combination thereof, the at least one base component is one selected from a group consisting of an organic base, a phosphate and a combination thereof, wherein the at least one acid component and the at least one base component conjugate with each other to form a buffer formulation, and the urease activity inhibitor is one selected from a group consisting of an ascorbic acid, a salt of the ascorbic acid and a combination thereof, wherein the buffer formulation stabilizes the activity of the urease activity inhibitor.

### BRIEF DESCRIPTION OF THE DRAWINGS

Other objectives, advantages and efficacies of the present invention will be described in detail below taken from the preferred embodiments with reference to the accompanying drawings.

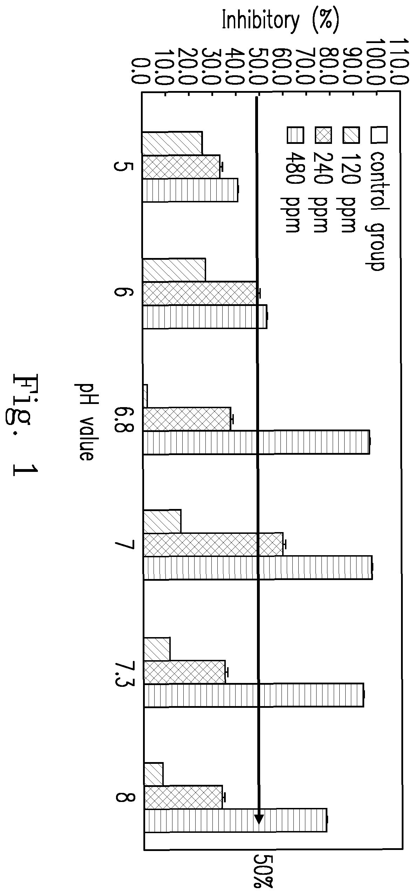

Fig. 1 is a histogram showing the inhibition of the urease hydrolysis of control group (containing the urease only) and experimental groups containing the urease and various concentrations (120 ppm, 240 ppm and 480 ppm) of ascorbic acid in the phosphate buffer solution measured at pH 5, 6, 6.8, 7.0, 7.3 and 8.

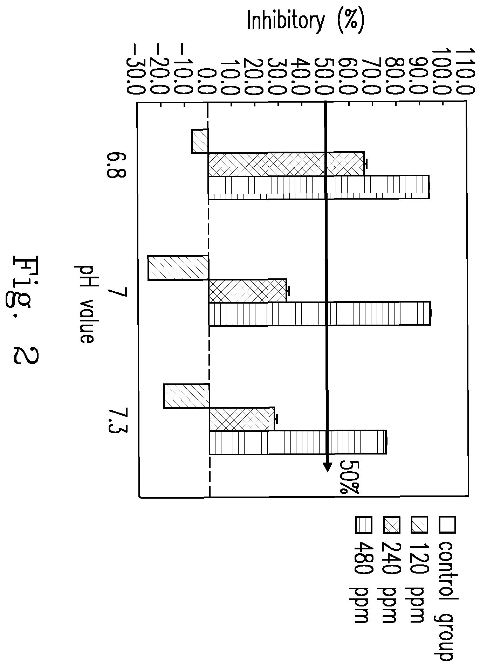

Fig. 2 is a histogram showing the inhibition of the urease hydrolysis of control group (containing the urease only) and experimental groups containing the urease and various concentrations (120 ppm, 240 ppm and 480 ppm) of ascorbic acid in the citrate buffer solution measured at pH 6.8, 7.0 and 7.3.

### DETAILED DESCRIPTION OF THE PREFERRED EMBODIMENTS

The buffer formulation composition provided in the present invention includes at least one acid component, at least one base component, and a urease inhibitor. Because the buffer formulation composition of the present invention can be applied to animal feed or nutritional supplements for humans or animals, and can also be added into soil or the urea fertilizer to reduce ammonia emissions and consumption of the urea fertilizer owing to the catalyzed hydrolysis of the urea fertilizer by the urease, the components used in the buffer formulation must be non-toxic to the organism and environmentally friendly. Therefore, the acid component is preferably an organic acid, the base component is preferably an organic base, and the urease activity inhibitor is ascorbic acid, the salts of the ascorbic acid or combinations thereof. In addition, as phosphoric acid and phosphates are widely used as food additives, therefore, they are also encompassed by the scope of the acid component and base component of the present invention.

The acid component has a conjugate relationship with the base component to form a buffer formulation, e.g., the buffer formulation may be formulated in an organic acid and its conjugate base, or an organic base and its conjugate acid. The pH value of the buffer formulation is determined by the dissociation constant (pKa) of the acid component and the ratio of the acid component to the base component. In general, the pH value is in the range of ±1 pKa. The pKa values of common organic acids, phosphoric acid, and ascorbic acid are shown in Table 1 (see Lab Manual for Zumdahl/Zumdahl's Chemistry 6th Edition). Among these acid components, phosphoric acid having a higher pKa value can work in an alkaline environment, and therefore it is the best choice for the acid component in the buffer formulation of the present invention.

| Organic acids | Number of carbon | pKa1 | pKa2 | pKa3 |
|---|---|---|---|---|
| (1) formic acid | 1 carbon atom | 3.75 | -- | -- |
| (2) acetic acid | 2 carbon atoms | 4.76 | -- | -- |
| (3) propionic acid | 3 carbon atoms | 4.88 | -- | -- |
| (4) butyric acid | 4 carbon atoms | 4.82 | -- | -- |
| (5) malic acid | 4 carbon atoms | 3.4 | 5.1 | -- |
| (6) fumaric acid | 4 carbon atoms | 3.02 | 4.38 | -- |
| (7) lactic acid | 3 carbon atoms | 3.83 | -- | -- |
| (8) citric acid | 6 carbon atoms | 3.13 | 4.76 | 6.4 |
| (9) ascorbic acid | 6 carbon atoms | 4.1 | 11.8 | -- |
| (10) phosphoric acid | 0 carbon atom | 2.12 | 7.21 | 12.32/12.66 |

The buffer formulation formed by the appropriate acid component and base component can have multiple pHs, so it can adapt to the changes in the gastrointestinal environment to have the urease activity inhibitor therein perform better in the gastrointestinal tract.

The acid component used in the present invention includes formic acid, acetic acid, propionic acid, butyric acid, malic acid, fumaric acid, lactic acid, citric acid and phosphoric acid. In a preferred embodiment, the acid component is the phosphoric acid or the citric acid. The base component used in the present invention is an organic base or phosphate, preferably, the organic base or the phosphate refers to an alkali metal salt or an alkaline earth metal salt. In a preferred embodiment, the phosphate is dipotassium hydrogen phosphate or disodium hydrogen phosphate, and the organic base is sodium citrate or potassium citrate.

The buffer formulation of the present invention may also include a plurality of organic acids and their conjugated bases, or a plurality of organic bases and their conjugated acids. The organic acid may also be combined with the phosphoric acid to regard as the acid component in the buffer formulation of the present invention, and the organic base may also be combined with the phosphate to regard as the base component in the buffer formulation of the present invention. Any buffer formulation that can be formulated as being suitable for gastrointestinal pH conditions is within the scope of the present invention.

The urease activity inhibitor used in the present invention is the ascorbic acid, the salts of the ascorbic acid or combinations thereof. Preferably, the salt of the ascorbic acid is one of a sodium salt, a potassium salt, a calcium salt, a magnesium salt or a combination thereof.

The ascorbic acid (L-ascorbic acid), also known as vitamin C, is a water-soluble and easily absorbed compound. The ascorbic acid is easily degraded in an environment where the pH is greater than 4. The buffer formulation of the present invention can steadily sustain the activity of ascorbic acid, the salts of the ascorbic acid, or a combination thereof in the environment at pH greater than 4, so as to achieve the goal of inhibiting the activity of urease in the soil or in the gastrointestinal tract of humans or animals, especially, as the environment is across acid and base.

In another aspect, the composition of the present invention can be used as a composition for inhibiting the activity of urease. For example, the composition may be an additive for nutritional supplements such as a formulated milk powder, an animal feed or a fertilizer, or it can be directly manufactured as a drink, which inhibits the activity of urease in the environment with a pH of 5-8, and preferably inhibits the activity of urease in the environment at pH of 6.8∼7.3.

According to the present invention, the above-mentioned composition includes at least one acid component, at least one base component and a urease activity inhibitor, wherein the at least one acid component and the at least one base component form a buffer formulation, and the buffer formulation enables the urease activity inhibitor to steadily maintain the activity in an environment across acid and base. In one embodiment, the urease activity inhibitor is distributed in the buffer formulation. In another embodiment, the buffer formulation is evenly mixed with the urease activity inhibitor.

In the present invention, the urease activity inhibitor is ascorbic acid, its sodium salt, potassium salt, calcium salt, magnesium salt or a combination thereof. In a preferred embodiment, the urease activity inhibitor is ascorbic acid, especially L-ascorbic acid. Preferably, the concentration of the ascorbic acid ranges from 120 ppm to 480 ppm, and more preferably 480 ppm. Of course, the concentration of the ascorbic acid of the present invention may be higher as long as it meets the recommended daily intake for the human body (up to about 2 g per day).

According to the present invention, the dosage form of the above-mentioned composition includes powder, particle, tablet, micron-particle, liquid, capsule or spray. The above-mentioned dosage form can also be designed as a delayed or extended-release formula such as delayed or extended-release tablet, delayed or extended-release micron-particle or delayed or extended-release capsule. It can also be manufactured as a drink for humans or animals, added into drinking water for humans or animals, or manufactured as a fertilizer spray used in the soil.

### Embodiments

### I. Experimental methods

### 1. Experiment of the inhibition of the activity of the urease (in phosphate buffer solution, pH 5.0)

1.1 Control group: 20 µL of the buffer solution and 25 µL of 50 ppm urease (U7752, Sigma-Aldrich) were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A (prepared by mixing 5g of phenol and 25mg of sodium nitroprusside and diluting the mixture to 500 mL with purified water) and 60 µL of Berthelot reagent B (prepared by mixing 2.5g of sodium hydroxide and 4.2 mL of sodium hypochlorite and diluting the mixture to 500 mL with purified water) were added into each well. The mixture was held in the dark at 37 °C for 30 minutes, and the absorbance was measured at 625 nm wavelength by an enzyme-linked immuno-sorbent assay (ELISA) reader. Each sample was measured at least in triplicate.
1.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### 2. Experiment of the inhibition of the activity of the urease (in phosphate buffer solution, pH 6.0)

2.1 Control group: 20 µL of the buffer solution and 25 µL of 50 ppm urease were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate.
2.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### 3. Experiment of the inhibition of the activity of the urease (in phosphate buffer solution, pH 6.8)

3.1 Control group: 20 µL of the buffer solution and 25 µL of 50 ppm urease were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate.
3.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### 4. Experiment of the inhibition of the activity of the urease (in phosphate buffer solution, pH 7.0)

4.1 Control group: 20 µL of the buffer solution and 25 µL of 50 ppm urease were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate.
4.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### 5. Experiment of the inhibition of the activity of the urease (in phosphate buffer solution, pH 7.3)

5.1 Control group: 20 µL of the buffer solution and 25 µL of 50 ppm urease were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate.
5.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### 6. Experiment of the inhibition of the activity of the urease (in phosphate buffer solution, pH 8.0)

6.1 Control group: Control group: 20 µL of the buffer solution and 25 µL of 50 ppm urease were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate.
6.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### 7. Experiment of the inhibition of the activity of the urease (in citric acid salt buffer solution, pH 6.8)

7.1 Control group: Control group: 20 µL of the buffer solution and 25 µL of 50 ppm urease were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate.
7.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### 8. Experiment of the inhibition of the activity of the urease (in citric acid salt buffer solution, pH 7.0)

8.1 Control group: Control group: 20 µL of the buffer solution and 25 µL of 50 ppm urease were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate.
8.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### 9. Experiment of the inhibition of the activity of the urease (in citric acid salt buffer solution, pH 7.3)

9.1 Control group: Control group: 20 µL of the buffer solution and 25 µL of 50 ppm urease were added and mixed in wells of a 96-well plate. After the mixture reacted at 37 °C for 5 minutes, 40 µL of 20 mM urea solution was added into the mixture. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate.
9.2 Ascorbic acid groups: 10 µL of the buffer solution, 10 µL of ascorbic acid solution and 25 µL of 50 ppm urease were added and mixed in wells of a 96-well plate. After the mixture reacted at 37 °C for 5 minutes, 40 µL of 20 mM urea solution was added into the mixture to make the final concentrations of the ascorbic acid as 120 ppm, 240 ppm and 480 ppm, respectively. After the mixture reacted at 37 °C for 10 minutes, 55 µL of Berthelot reagent A and 60 µL of Berthelot reagent B were added into each well. The mixture was held in the dark at 37 °C for 30 minutes, and the absorbance was measured at 625 nm wavelength by the ELISA reader. Each sample was measured at least in triplicate. The inhibition % of the activity of the urease=100 - ((the absorbance of the sample/the absorbance of the control group) × 100).

### II. Results

### 1. Experimental example 1

In this embodiment, the phosphate buffer solution (PBS) was formed by a phosphoric acid and a phosphate, and the inhibition of the urease activity of the control group and the experimental groups (containing 120 ppm, 240 ppm and 480 ppm of ascorbic acid) are measured at various pH values.

Please refer to Table 2 and Fig. 1, which show the inhibitory data of the urease hydrolysis of control group (containing the urease only) and experimental groups containing the urease and various concentrations (120 ppm, 240 ppm and 480 ppm) of ascorbic acid. It can be observed from Table 2 that inhibition of the urea hydrolysis is positively correlated with the concentration of the ascorbic acid. While taking 50% inhibition as a comparison indicator, the inhibition of the group containing 480 ppm ascorbic acid was >50% at pH 6.0-8.0, and the inhibitory thereof was >93% at pH 6.8-7.3, which show that the group containing 480 ppm ascorbic acid had a significant inhibitory effect with respect to the control group.

**Table 2**

| | | | | | | |
|---|---|---|---|---|---|---|
| pH value | 5 | 6 | 6.8 | 7 | 7.3 | 8 |
| Control group | 0.0 | 0.0 | 0.0 | 0.0 | 0.0 | 0.0 |
| 120 ppm | 25.7 | 26.7 | 2.0 | 16.1 | 11.3 | 8.1 |
| 240 ppm | 33.1 | 49.0 | 37.3 | 59.4 | 34.6 | 33.3 |
| 480 ppm | 40.5 | 52.9 | 96.5 | 97.2 | 93.6 | 77.7 |

### 2. Experimental example 2

In this embodiment, the citrate buffer solution (CBS) was formed by a citric acid and a citric acid salt, and the inhibition of the urease activity of the control group and the experimental groups (containing 120 ppm, 240 ppm and 480 ppm of ascorbic acid) are measured at various pH values.

Please refer to Table 3 and Fig. 2, which show the inhibition of the urease hydrolysis of control group (containing the urease only) and experimental groups containing the urease and various concentrations (120 ppm, 240 ppm and 480 ppm) of ascorbic acid. It can be observed from Table 3 and Fig. 2 that inhibition of the urea hydrolysis is positively correlated with the concentration of the ascorbic acid. While taking 50% inhibition as a comparison indicator, the inhibition of the group containing 480 ppm ascorbic acid was >50% at pH 6.8-7.3, and the inhibition thereof was >90% at pH 6.8-7.0, which shows that the group containing 480 ppm ascorbic acid had a significant inhibitory effect with respect to the control group.

**Table 3**

| | | | |
|---|---|---|---|
| pH value | 6.8 | 7.0 | 7.3 |
| Control group | 0.0 | 0.0 | 0.0 |
| 120 ppm | -6.8 | -25.2 | -19.7 |
| 240 ppm | 66.0 | 32.6 | 27.1 |
| 480 ppm | 93.5 | 93.6 | 75.3 |

With respect to the optical density (OD) values in each pH environment, a higher optical density value indicates a higher concentration of the measured ammonia. In the environment with pH 5-8, the optical density value of the control group was used as a comparative indicator. It was observed that ascorbic acid has the inhibitory effect on the urease hydrolysis in the environment with pH 5-8, especially the inhibitory effect is over 90% in the environment with pH 6.8-7.3.

Because the composition of the present invention does not contain any drug component, it can be used without the doubt in drug resistance, drug-residue and the concern for food safety. When the composition of the present invention is used as an additive of human or animal nutritional supplements, it has the effect of inhibiting the urease activity.

It is understood, that this invention is not limited to the particular embodiments disclosed, but is intended to cover all modifications which are within the spirit and scope of the invention as defined by the appended claims, the above description, and/or shown in the attached drawings.

## Claims

1. A composition for use in a method for inhibiting an activity of a urease, the composition **characterized by** comprising:
at least one acid component being one selected from a group consisting of an organic acid, a phosphoric acid and a combination thereof;
at least one base component being one selected from a group consisting of an organic base, a phosphate and a combination thereof, wherein the at least one acid component and the at least one base component conjugate with each other to form a buffer formulation; and
a urease activity inhibitor being one selected from a group consisting of an ascorbic acid, a salt of the ascorbic acid and a combination thereof, wherein the buffer formulation steadily sustains the activity of the urease activity inhibitor.

2. The composition for use as claimed in Claim 1, **characterized in that** the buffer formulation has multiple pHs in an environment ranged from pH 5 to pH 8, and the urease is in one selected from a group consisting of a human being, a soil and a non-human animal.

3. The composition for use as claimed in Claim 1, **characterized in that** the buffer formulation steadily sustains the activity of the ascorbic acid when pH > 4.

4. A composition for inhibiting an activity of a urease, **characterized by** comprising:
at least one acid component being one selected from a group consisting of an organic acid, a phosphoric acid and a combination thereof;
at least one base component being one selected from a group consisting of an organic base, a phosphate and a combination thereof, wherein the at least one acid component and the at least one base component conjugate with each other to form a buffer formulation; and
a urease activity inhibitor being one selected from a group consisting of an ascorbic acid, a salt of the ascorbic acid and a combination thereof, wherein the buffer formulation steadily sustains the activity of the urease activity inhibitor.

5. The composition as claimed in Claim 4, **characterized in that** the composition is added into one of a nutritional supplement and a drink, wherein the nutritional supplement is one of a formulated milk powder and an animal feed.

6. The composition as claimed in Claim 4, **characterized in that** the organic acid is one selected from a group consisting of a formic acid, an acetic acid, a propionic acid, a butyric acid, a malic acid, a fumaric acid, a lactic acid and a citric acid, and either of the organic base and the phosphate is one of an alkali metal salt and an alkaline earth metal salt.

7. The composition as claimed in Claim 6, **characterized in that** the at least one acid component is one of the phosphoric acid and the citric acid, the phosphate is one of a dipotassium hydrogen phosphate and a disodium hydrogen phosphate, and the organic base is one of a sodium citrate and a potassium citrate.

8. The composition as claimed in Claim 4, **characterized in that** the buffer formulation is evenly mixed with the urease activity inhibitor.

9. The composition as claimed in Claim 4, **characterized in that** the buffer formulation has multiple pHs in an environment ranged from pH 5 to pH 8.

10. The composition as claimed in Claim 4, **characterized in that** the ascorbic acid has a concentration range from 120 ppm to 480 ppm.

11. The composition as claimed in Claim 4, **characterized in that** the salt of the ascorbic acid is one selected from a group consisting of a sodium salt, a potassium salt, a calcium salt, a magnesium salt and a combination thereof.

12. The composition as claimed in Claim 4, **characterized in that** the composition inhibits the activity of the urease in an environment at pH of 5-8.

13. The composition as claimed in Claim 12, **characterized in that** the composition inhibits the activity of the urease in an environment at pH of 6.8-7.3.

14. The composition as claimed in Claim 4, **characterized in that** the composition is manufactured as one being selected from a group consisting of a powder, a particle, a tablet, a micron-particle, a liquid and a capsule.

15. The composition as claimed in Claim 4, **characterized in that** the ascorbic acid is an L-ascorbic acid.
